# EUROPÄISCHE PATENTSCHRIFT

(11) **EP 2 268 264 B1**
(45) Veröffentlichungstag und Bekanntmachung des Hinweises auf die Patenterteilung: **24.10.2018**
(21) Anmeldenummer: 09728884.9
(22) Anmeldetag: 03.04.2009
(51) Int. Cl.: A61K 9/00, A61L 27/00

(54) **QUERVERNETZTE POLYMERMATRIX, INSBESONDERE ZUR VERABREICHUNG VON WIRKSTOFFEN**
CROSS-LINKED POLYMER MATRIX, IN PARTICULAR FOR ADMINISTERING ACTIVE INGREDIENTS
MATRICE POLYMÈRE RÉTICULÉE, EN PARTICULIER POUR L'ADMINISTRATION DE PRINCIPES ACTIFS

(30) Priorität: 03.04.2008 DE 102008016998
(43) Veröffentlichungstag der Anmeldung: 05.01.2011
(73) Patentinhaber: Universität Regensburg, 93053 Regensburg (DE)
(72) Erfinder: GÖPFERICH, Achim, 93161 Sinzing (DE)
(74) Vertreter: Ahrens, Gabriele
(86) Internationale Anmeldenummer: PCT/EP2009/054023
(87) Internationale Veröffentlichungsnummer: WO 2009/121961

(56) Entgegenhaltungen:
- WO-A-02/076429
- WO-A-2004/104076
- WO-A-2005/105167
- WO-A1-2007/135114
- MASAYUKI HARA ET AL: "Noninvasive detachment of cells on cells", MATERIALS SCIENCE AND ENGINEERING: C, vol. 17, no. 1-2, 1 November 2001 (2001-11-01), pages 107-112, XP055184304, ISSN: 0928-4931, DOI: 10.1016/S0928-4931(01)00317-4

## Beschreibung

Gegenstand der vorliegenden Erfindung sind Polymermatrizes, die als Wirkstoffträger eingesetzt und im menschlichen oder tierischen Körper lokal angewendet werden können. Insbesondere betrifft die vorliegende Erfindung derartige Polymermatrizes, die am Einsatzort im Körper über einen Zeitraum von mehreren Stunden oder Tagen einen inkorporierten Wirkstoff verzögert freisetzen und sich dabei in der Körperflüssigkeit auflösen, sodass sie nicht mehr entfernt werden müssen.

Die Einbettung in eine Matrix ist ein anerkanntes Verfahren, um Wirkstoffe über einen längeren Zeitraum im Körper freizusetzen. Voraussetzung dafür ist ein geeignetes Netzwerk, um die Diffusion innerhalb der Matrix zu verzögern oder sogar ganz zu verhindern. Um eine Freisetzung über mehrere Stunden zu gewährleisten, werden Hydrogele häufig eingesetzt.

Hydrogele, zum Beispiel aus gelbildenden Polysacchariden wie Alginaten, sind allgemein bekannt und in der Literatur zahlreich beschrieben.

Alginate sind Salze der Alginsäure, die u. a. Guluron-Säure als Monomere enthalten.
Die einzelnen Polymerketten lassen sich mit Hilfe von mehrwertigen Kationen quer vernetzen, wobei die Kationen mit der Guluron-Säure von verschiedenen Polymerketten ionische Bindungen ausbilden.

Beispielsweise beschreibt DE 10 2004 019 241 A1 injizierbare vernetzte und unvernetzte Alginate und deren Verwendung in der Medizin und in der ästhetischen Chirurgie als Füllmaterial zur Volumen- und Defektauffüllung, zum Beispiel zur Aufpolsterung von Falten. Es wird beschrieben, dass das injizierte Alginat bei Bedarf am Einsatzort wieder aufgelöst werden kann, indem ein weiteres Mittel injiziert wird, das die Kationen aus der Bindung mit den Polymerketten drängt und somit die Quervernetzung auflöst. Das injizierte Alginat als solches ist jedoch nicht selbstauflösend.

DE 103 23 794 A1 betrifft ein Verfahren zur Herstellung von großformatigen, insbesondere relativ dicken (zum Beispiel 1 cm oder mehr) Formkörpern auf Alginatbasis. Diese Formkörper weisen eine hohe Nassfestigkeit auf und können in dünne Schichten geschnitten als kosmetische Hautauflage oder medizinische Wundauflage eingesetzt werden. Weiter werden sie für orale, buccale oder nasale Anwendungen eingesetzt.
Es handelt sich hierbei um haltbare, nur schwer lösliche Gele.

Ein Verfahren zur kontrollierten Quervernetzung von Alginaten ist in DE 697 07 475 T2 beschrieben. Es wird vorgeschlagen, durch Einstellung des Gehalts an Quervernetzungen im Alginat, dessen Eigenschaften wie Viskosität, Elastizität, Festigkeit etc. zu kontrollieren.

DE 37 21 163 A1 betrifft ein Trägermaterial für Augenarzneimittel. Als Trägermaterial wird ein Alginatgel vorgeschlagen. Die Gelbildung, das heißt Quervernetzung, erfolgt hier in vivo, indem das Alginat und das Vernetzungsmittel separat am Auge appliziert werden und vor Ort am Auge das Gel ausgebildet wird.

WO 2007/135114 A1 beschreibt Mikrokapseln aus Hydrogelen, wobei als gelbildende Polymerkomponente eine Mischung aus einem anionischen Polysaccharid, zum Beispiel Alginat, und einem Oligosaccharidderivat von Chitosan eingesetzt wird. Die erhaltenen Kapseln lassen sich auch als Wirkstoffträger einsetzen. Eine Selbstauflösung ist nicht vorgesehen.

Damit Hydrogele als Wirkstoffträger den Wirkstoff über einen längeren Zeitraum im Körper freisetzen können, müssen sie wie vorstehend auch im Stand der Technik beschrieben, quervernetzt werden. Andernfalls würden sie im wässrigen (physiologischen) Milieu stark aufquellen und ihre Barrierefunktion verlieren mit der Folge, dass der Wirkstoff innerhalb kürzester Zeit freigesetzt werden würde. Eine verzögerte, allmähliche Freisetzung z.B. über Stunden, ist mit nicht quervernetzten Hydrogelen nicht gewährleistet.

Es sind auch Kollagen- und Gelatinegele bekannt, die mit reaktiven Substanzen wie Glutardialdehyd kovalent vernetzt werden, um die Quellung einzuschränken. Die Freisetzung aus diesen Gelen erfolgt über mehrere Stunden. Allerdings ist die Herstellungsmethode solcher Gele für Protein-Wirkstoffe auf Peptidbasis nur wenig geeignet, da diese während der Quervernetzung beschädigt werden könnten. Außerdem sind solche kovalent-quervernetzten Gele nur begrenzt erodierbar und verbleiben damit über die Therapie hinaus am Applikationsort, was zur Folge haben kann, dass man sie entfernen muss, um Nebenwirkungen zu vermeiden.

WO 2007/135114 A1 beschreibt Hydrogele, die Wirkstoffe verkapsuliert enthalten. Eine Selbstauflösung dieser Hydrogele am Applikationsort ist nicht vorgesehen.
Gemäß einer Herstellungsmethode wird in einem ersten Schritt eine Polymerlösung der zu vernetzenden Polymere (Alginate) hergestellt, der Lösung in einem zweiten Schritt eine inaktive Form eines Kalziumsalzes wie Kalziumcarbonat oder ein Ca-EDTA-Komplex zugesetzt, und in einem dritten Schritt der Lösung mit inaktiven Ca-Ionen ein Mittel zugesetzt, das die Ca-Salze hydrolisiert und somit die Ausbildung des Hydrogels auslöst.

In Masayuki Hara et al.: "Noninvasive detachment of cells on cells", Material Science and Engineering: C, Band 17, Nr. 1-2, 1. November 201, Seiten 107 bis 112 wird die Durchführung einer Zellkultur auf einer Collagenschricht beschrieben. Hierzu ist die Collagenschicht auf eine Ca-Alginatschicht aufgebracht, die wiederum auf einem Membranfilter aufgebracht ist. Diese Publikation betrifft insbesondere die Loslösung der mit einer Zellkultur versehenen Collagenschicht von dem Membranfilter nach Inkubation.

Dem Fachmann sind drei Möglichkeiten der Bioerosion von Hydrogelen bekannt: chemische Hydrolyse kovalenter Bindungen, enzymatischer Abbau und Auflösen in einem Lösungsmittel, wobei das Lösungsmittel (wie z. B. Wasser) die Interaktionen zwischen den Polymerketten aufhebt.

Im Falle der kovalent quervernetzten Gele kommt als Mechanismen nur ein chemischer oder enzymatischer Abbau in Betracht. Allerdings sind gängige kovalente Bindungen gegenüber einfacher chemischer Hydrolyse normalerweise recht stabil; im lebenden Organismus kommen als Biokatalysatoren eine Reihe von Enzymen in Betracht, welche diese Reaktion auch unter physiologischen Bedingungen ablaufen lassen. Kovalent quervernetzte Hydrogele können also im lebenden Organismus abgebaut werden, allerdings dauert dieser Vorgang entweder sehr lange oder er ist an das Vorhandensein der entsprechenden Enzyme am Applikationsort gebunden.

Für die Zwecke der vorliegenden Erfindung sind die vorstehend genannten quervernetzten Hydrogele nicht geeignet. Die kovalent quervernetzten Hydrogele sind ungeeignet, da sich erfindungsgemäß die Matrizes zum einen innerhalb weniger Stunden auflösen sollen, zum anderen aber auch an leicht zugänglichen Körperregionen anwendbar sein sollen, wie zum Beispiel der Augenoberfläche, an der nur wenige Enzyme vorliegen.
Daher kommt als geeigneter Erosionsmechanismus nur eine Auflösung der Matrizes in Betracht. Normalerweise versteht man unter dem Begriff Auflösung einen allmählichen "Abbau" eines Festkörpers nach dessen Kontakt mit einem Lösungsmittel, wobei als Lösungsmittel für die vorliegende Erfindung aufgrund der Anwendung vorzugsweise wässrige Systeme in Betracht kommen. Allerdings lösen sich quervernetzte Gele unter solchen Bedingungen nicht, sondern sie quellen nur. Unter einer Quellung versteht man eine Vergrößerung des Abstands zwischen den einzelnen Molekülen des Gelbildners (wie z. B. Polymerketten) durch Einlagerung von Lösungsmittelmolekülen. Dabei können sich Gele, wie die vorstehend genannten Alginate nicht im herkömmlichen Sinne auflösen, da die Gelbildnermoleküle an den Knotenpunkten zusammengehalten werden. Unter Knotenpunkten versteht man die Stellen im Gel, an denen die Moleküle des Gelbildners durch ein quervernetzendes Agens zusammengehalten werden. Um ein quervernetztes Hydrogel aufzulösen, muss dieses quervernetzende Agens entfernt oder inaktiviert werden, um so den Knotenpunkt aufzulösen.

Sieht man den Knotenpunkt als eine Kombination aus di- oder multivalentem Ligand (= quervernetzendes Agens) und Rezeptor (= Bindungsstelle am Gelbildner), sind folgende Möglichkeiten, oder Kombinationen davon, denkbar um ein quervernetzendes Agens zu inaktivieren und damit ein solches Gel wieder aufzulösen :
*a) Verringerung der Affinität des Liganden zum Rezeptor durch einen zweiten, externen Rezeptor*
   Im quervernetzten Gel bindet der Ligand mit einer bestimmten Affinität an die Rezeptoren und verbindet somit mehrere Moleküle des Gelbildners. Durch Zugabe eines zweiten, löslichen Rezeptors (der nicht mit dem am Gelbildner fixierten identisch sein muss), wird der Ligand aus seiner Bindung herausgelöst. Voraussetzung dabei ist eine höhere Affinität des Liganden zu dem löslichen Rezeptor als zu dem am Gelbildner fixierten.
*b) Verringerung der Affinität des Liganden zum Rezeptor durch Veränderung des Liganden*
   Durch einen äußeren Schalter, z. B. eine Änderung des pH-Wertes oder der Temperatur, ändert sich die Struktur des Liganden selbst derart, dass er seine Affinität zum Rezeptor am Gelgerüst verliert. Dadurch wird die Bindung zwischen Ligand und Rezeptor gelöst.
*c) Verringerung der Affinität des Liganden zum Rezeptor durch Veränderung des Rezeptors*
   Durch einen äußeren Schalter, z. B. eine Änderung des pH-Wertes oder der Temperatur, ändert sich die Struktur des Rezeptors derart, dass der Ligand seine Affinität zum Rezeptor verliert und folglich die Bindung zwischen Ligand und Rezeptor gelöst wird.
*d) Ersatz des Liganden durch eine monovalente Form*
   Durch Zugabe eines zweiten Liganden, der aufgrund seiner Struktur allerdings nur an jeweils einen Rezeptor binden kann (= monovalent), wird im quervernetzten Gel der multivalente Ligand aus seiner Bindung verdrängt und die fixierten Rezeptoren blockiert.
   Nachstehend ist eine schematische Darstellung der genannten Möglichkeiten zur Inaktivierung gezeigt:

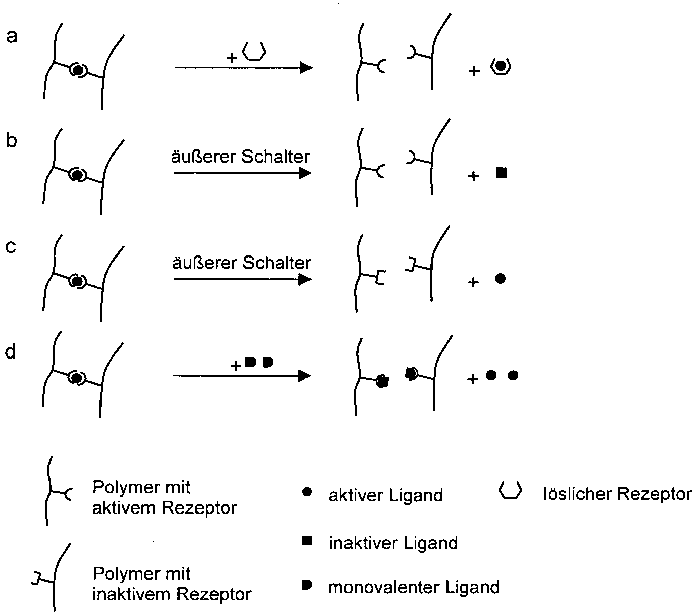

Der vorliegenden Erfindung liegt damit die Aufgabe zugrunde, eine quervernetzte Polymermatrix bereitzustellen, die als Wirkstoffträger mit verzögerter Wirkstofffreisetzung verwendet werden kann. Insbesondere war es Aufgabe, eine derartige Polymermatrix bereitzustellen, die sich am Applikationsort, zum Beispiel am oder im Körper eines Menschen oder Tieres, vergleichsweise schnell, insbesondere innerhalb von Stunden, von selbst auflöst.
Gemäß einem Aspekt der Erfindung soll die Auflösung unabhängig von der Enzymmenge erfolgen, die am Applikationsort vorliegt. Insbesondere soll die vorliegende Erfindung auch für leicht zugängliche Körperregionen angewendet werden können, an denen nur wenige Enzyme vorliegen, wie zum Beispiel der Augenoberfläche.
Gemäß einem weiteren Aspekt war es Aufgabe der vorliegenden Erfindung, eine Polymermatrix bereitzustellen, die als selbstauflösendes Insert/Implantat zur Applikation in oder an einem Körper eingesetzt werden kann.

Weiter war es Aufgabe der vorliegenden Erfindung, eine Polymermatrix oder ein daraus hergestelltes Insert bereitzustellen, die als Wirkstoffträger einen Wirkstoff über einen längeren Zeitraum im Körper freisetzen kann.

Die vorstehende Aufgabe wird gelöst durch eine Polymermatrix gemäß Anspruch 1, wobei die Unteransprüche bevorzugte Ausgestaltungsmerkmale der erfindungsgemäßen Polymermatrix betreffen.

Die erfindungsgemäße Polymermatrix basiert auf einem aus einem ternären System aufgebauten Hydrogel, dessen drei Komponenten ein gelbildendes Polymer, ein quervernetzendes Agens und ein auflösendes Agens sind, dessen Aufbau nachstehend schematisch dargestellt ist:

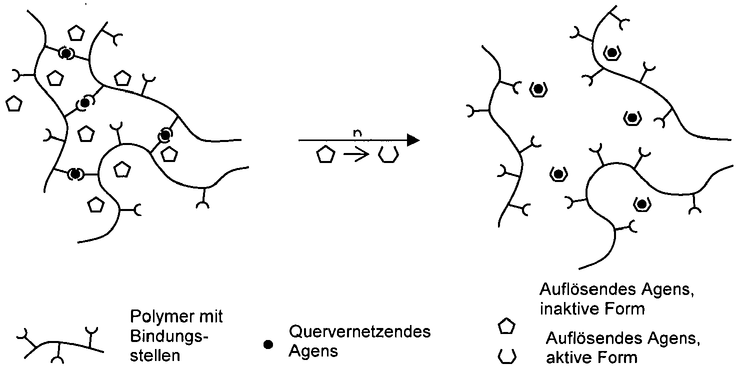

Polymere sind langkettige, aus sich wiederholenden Einheiten (sogenannten Monomeren) aufgebaute Makromoleküle. Ein für die vorliegende Erfindung geeignetes Polymer trägt an seinen Monomeren in regelmäßiger oder unregelmäßiger Abfolge Bindungsstellen für das quervernetzende Agens. Dieses kann aufgrund seiner Struktur von mindestens zwei solchen Bindungsstellen gebunden werden und verbindet folglich zwei oder mehr Polymerketten. Auf diese Weise bilden das Polymer und das quervernetzende Agens das Gelgerüst der erfindungsgemäßen Polymermatrix aus.
Das auflösende Agens ist darin zunächst in einer inaktiven Form verteilt, ohne an einer der anderen Komponenten fixiert zu sein. Um ein solches erfindungsgemäßes Hydrogel wieder aufzulösen, wird das auflösende Agens durch einen äußeren Stimulus (z.B. eine Änderung des pH-Wertes) aktiviert.

Bei der Auflösung des erfindungsgemäßen Hydrogels handelt es sich demnach um eine Kombination der oben beschriebenen Möglichkeiten a) und c).

Im Unterschied zum oben beschriebenen Fall a) wird das auflösende Agens (vorstehend in a) der "lösliche Rezeptor") nicht zugegeben, sondern durch einen äußeren Stimulus wie in Möglichkeit c) beschrieben, aktiviert. Im Unterschied zu dem "Rezeptor" in Fall c) liegt erfindungsgemäß das auflösende Agens frei im Gel beweglich vor.

Wird die Affinität des auflösenden Agens entweder zu dem quervernetzenden Agens oder dessen Bindungsstellen am Polymer durch einen äußeren Einfluss geändert, wird das auflösende Agens entweder an das quervernetzende Agens oder dessen Bindungsstelle am Polymer binden und somit einen Knotenpunkt auflösen.
Folglich wird das Gelgerüst langsam zerstört und die einzelnen Komponenten gehen in Lösung.

Beispiele für für die vorliegende Erfindung geeignete, gelbildende Polymere sind Polysaccharide, die als eines ihrer Monomere Guluronsäure enthalten.
In diesen Polysacchariden wirkt die Guluronsäure als Bindungsstelle für das quervernetzende Agens. Dazu muss die Guluronsäure entlang der Polymerkette in Blöcken von mindestens zwei Einheiten Länge angeordnet sein. Diese Blöcke bilden aufgrund ihrer Konformation die beschriebene Bindungsstelle für das quervernetzende Agens.

Ein Beispiel für derartige guluronsäure-haltige Polysaccharide sind Alginate, die neben der Guluronsäure noch deren Isomer Mannuronsäure als weiteres Monomer enthalten. Gemäß einer bevorzugten Ausführungsform der Erfindung wird Natriumalginat als gelbildendes Polymer verwendet.

Für die vorliegende Erfindung sollte der Gehalt an Guluronsäure in dem gelbildenden Polymer wie z.B. dem Alginat, vorzugsweise zwischen 30% (m/m) und 90% (m/m) liegen, und insbesondere zwischen 35% (m/m) und 75% (m/m). Beispiele für quervernetzende Agentien, die an die Guluronsäureeinheiten eines solchen Polymers binden können, sind mehrwertige Kationen.
Diese werden von den Guluronsäureblöcken verschiedener Polysaccharidketten komplexiert, so dass ein quervernetztes Gelgerüst entsteht.
Wegen seiner geringen Toxizität wird dabei Ca²⁺ bevorzugt eingesetzt. Die Stoffmenge an Calciumionen sollte zwischen der Hälfte und dem fünffachen des Polysaccharidgehalts liegen.
Nach Bedarf und Anwendung können auch andere Kationen wie z. B. Fe^{2+/3+}, Al³⁺, Zn²⁺ oder weitere an sich aus der Literatur hierfür bekannte Kationen eingesetzt werden. Die zu verwendende Stoffmenge an Kationen kann dabei entsprechend der Stoffmenge an Calciumkationen gewählt werden.

Auflösende Agentien im Sinne der Erfindung, die ein solches über mehrwertige Kationen quervernetztes Hydrogel wieder auflösen können, sind beispielsweise Komplexbildner, die mit mehrwertige Kationen stabile Komplexe bilden.

Die Stabilität eines Komplexes ist zum einen abhängig von der Art des mehrwertigen Kations und zum anderen von äußeren Einflüssen wie dem vorliegenden pH-Wert. Als äußerer Stimulus kann also der pH-Wert dienen, wobei z. B. bei niedrigen pH-Werten das mehrwertige Kation vom Polymer gebunden wird, bei neutralen bis alkalischen pH-Werten aber vom Komplexbildner
Ein Beispiel für einen solchen Komplexbildner ist Na₂-EDTA (Dinatrium-Ethylendiaminacetat), das in der Lage ist, mehrwertige Kationen wie z. B. Ca²⁺-Ionen, aus der Guluronsäure-Komplexierung herauszulösen.

Das auflösendem Agens sollte in der Polymermatrix im äquimolaren Verhältnis zum enthaltenen quervernetzenden Agens, bzw. zu den Bindungsstellen für dieses, enthalten sein.
Jedoch kann zur Steuerung der Erosion bzw. Abbaugeschwindigkeit die Stoffmenge an auflösendem Agens in der Polymermatrix variiert werden, z.B. auf die Hälfte reduziert oder bis zum Dreifachen gesteigert werden (jeweils bezogen auf die Stoffmenge des quervernetzenden Agens).

Das auflösende Agens kann in der Polymermatrix homogen oder, nach Bedarf, an ausgewählten Bereichen verteilt vorliegen. In der Regel ist eine homogene Verteilung bevorzugt.

Die Art des Wirkstoffes, der in die Polymermatrix inkorporiert sein kann, unterliegt prinzipiell keinen Beschränkungen, solange er mit der Polymermatrix verträglich ist.
Insbesondere eignet sich die erfindungsgemäße Matrix für die Applikation von Wirkstoffen wie Arzneimittel, die ein Protein oder Peptid sind. Beispiele hierfür sind ein Wachstumgsfaktor, Zytokin, epidermaler Wachstumsfaktor, etc.
Es können auch Wirkstoffe eingesetzt werden, die mit der Polymermatrix ionische Interaktionen ausbilden.

Es zeigen
- **Figur 1**: ein Diagramm mit dem Auflösungsverhalten der Polymermatrix nach Beispiel 1,
- **Figur 2**: den Aufbau einer Polymermatrix nach Beispiel 2,
- **Figur 3**: ein Diagramm des Auflösungsverhaltens der Polymermatrix nach Figur 2;
- **Figur 4**: schematisch den Aufbau einer Polymermatrix gemäß Beispiel 3; und
- **Figur 5**: das Diagramm des Aufösungsverhaltens der Polymermatrix gemäß Figur 4.

Nachstehend wird die vorliegende Erfindung, deren Herstellung und Anwendung näher erläutert, gegebenenfalls zur besseren Anschaulichkeit unter Bezugnahme auf eine bevorzugte Ausführungsform, wobei das auflösende Agens ein Komplexbildener, wie insbesondere EDTA, das quervernetzende Agens Calciumkation und das gelbildende Polymer Na-Alginat ist.

Natriumalginat kann bereits in der Kälte leicht mit Ca²⁺-Ionen quervernetzt werden, so das ein einzuarbeitender Wirkstoff, wie insbesondere Protein/Peptid, in seiner Stabilität nicht beeinflusst wird. Die Temperatur kann dazu in einem großen Bereich variieren, wobei sie nach unten hin durch den Gefrierpunkt der entsprechenden Lösung begrenzt wird; nach oben wird die Grenze durch die thermische Stabilität des einzuarbeitenden Wirkstoffes gesetzt.

Bei der Quervernetzung wird ein Ca²⁺-Ion von jeweils zwei aufeinanderfolgenden Guluronsäureeinheiten zweier Polysaccharidketten komplexiert und auf diese Weise mehrere Ketten aneinander gebunden. Das entstehende quervernetzte Hydrogel ist formstabil mit elastischen Eigenschaften, wie bei einem kovalent quervernetzten Gel.

Zwei bekannte Methoden für die Herstellung eines quervernetzten Alginatgels werden nachstehend beschrieben:
1. Einfaches Eintauchen von Na-Alginatgel, das ein nichtvernetztes Gel ist, in eine Ca²⁺-enthaltende Lösung, so dass die Ca²⁺-Ionen in das Gel hinein diffundieren können und die einwertigen Natriumkationen aus ihrer Verbindung verdrängen.
   Selbstverständlich ist es auch möglich, die das mehrwertige Kation enthaltende Lösung, dem Na-Alginatgel zuzusetzen.
2. Durch die sogenannte "innere Gelbildung", wobei die Ca²⁺-Ionen innerhalb der Natriumalginatlösung freigesetzt werden und so die Polysaccharidketten von innen heraus quervernetzen. Dazu wird entweder ein schwerlösliches Calciumsalz oder ein Komplexbildner eingesetzt, der die Ca²⁺-Ionen nur langsam abgibt.

Um ein quervernetztes Calciumalginatgel wieder aufzulösen, müssen die Ca²⁺-Ionen entfernt und durch einwertige Kationen, z.B. Na⁺-Ionen, ersetzt werden. Am leichtesten ist das möglich durch einfache Diffusion der einwertigen Kationen, allerdings dauert dieser Vorgang normalerweise sehr lange und erfordert einen Überschuss an einwertigen Kationen.

Zur Beschleunigung der Auflösung können die Ca²⁺-Ionen durch Komplexbildner mit erhöhter Affinität gegenüber Ca²⁺-Ionen gebunden werden, so dass sie nicht mehr zur Quervernetzung zur Verfügung stehen. Voraussetzung dafür ist, dass der neu ausgebildete Komplex ausreichend stabil ist, um das Calciumion aus der Bindung an die Guluronsäureblöcke zu lösen. Das ist normalerweise bei Komplexbildnern der Fall, die mit mehrwertigen Kationen sehr stabile Komplexe eingehen (logarithmische Komplexbildungskonstanten im Bereich von etwa 7 bis 36, je nach Kation).

Problem bei diesem Vorgehen ist, dass man das quervernetzte Gel in eine Lösung des Komplexbildners tauchen muss, was für biologische Anwendungen ausscheidet.
Erfindungsgemäß wird dieses Problem gelöst, indem der Komplexbildner als auflösendes Agens in inaktiver Form, aber schaltbar aktivierbar, dem quervernetzten Hydrogel zugesetzt ist. Mit anderen Worten, in der erfindungsgemäßen Polymermatrix liegt das auflösende Agens bereits in inaktivierter Form inkorporiert vor.

Ein Beispiel für einen derartigen "schaltbaren" Komplexbildner ist EDTA, das bei niedrigen pH-Werten stabile Komplexe mit einwertigen Kationen, wie dem Natriumkation, bildet, jedoch bei einem neutralen oder basischen pH-Wert im Bereich von 7 und mehr wie er z. B. in Körperflüssigkeiten vorliegt, eine größere Komplexbildungsaffinität mit mehrwertigen Kationen wie Ca²⁺ hat.

Bei der Herstellung der erfindungsgemäßen Polymermatrix muss darauf geachtet werden, dass der als auflösendes Agens eingesetzte Komplexbildner die Quervernetzung der Polymerketten durch die mehrwertigen Kationen nicht behindert. Der zur Auflösung der Matrix führende Komplex, d.h. die Aktivierung des auflösenden Agens, darf also erst nach Applikation der Polymermatrix an den Anwendungsort entstehen.

Dies kann zum Beispiel durch eine räumliche Trennung von Komplexbildner und Kation während der Herstellung geschehen, die dann nach der Applikation durch die vorhandene Körperflüssigkeit wieder rückgängig gemacht wird wie in den Ausführungsformen gemäß Beispielen 2 und 3.

Hierzu kann der Komplexbildner als Feststoff ungelöst in die Polymermatrix inkorporiert sein und in dieser verteilt vorliegen. In Kontakt mit einer Körperflüssigkeit geht der Feststoff allmählich in Lösung und der Komplexbildner beginnt zu wirken.

Die Polymermatrix kann auch als Schichtsystem ausgebildet sein.

In diesem Fall kann die räumliche Trennung dadurch bewirkt werden, dass das auflösende Agens nur einem Teil der Schichten zugesetzt wird, während der Rest der Schichten kein auflösendes Agens enthält.

Eine bevorzugte Ausführungsform der räumlichen Trennung in einem Schichtsystem zeigt Beispiel 2, wobei die das auflösende Agens enthaltene Schicht unvernetzt ist und zwischen quervernetzen Schichten angeordnet ist.

Eine andere, weitaus elegantere Methode ist die vorübergehende Absenkung der Stabilität des Komplexes aus mehrwertigen Kation und Komplexbildner, damit die Ionen dann leichter von den Guluronsäureblöcken gebunden werden können. Auch diese Herabsetzung der Komplexbildungskonstante muss reversibel sein, was zum Beispiel durch Benutzung von EDTA als Komplexbildner gewährleistet wird.
Die Herstellung einer solchen Polymermatrix kann analog zu der aus der Literatur bekannten Methode der inneren Gelbildung von Alginaten erfolgen. Dabei wird durch ein hydrolyselabiles, säure-abspaltendes Agens der pH-Wert einer Lösung aus gelbildenden Polymer verringert. Durch den niedrigen pH-Wert werden Ca²⁺-Ionen aus dem Ca-EDTA-Komplex freigesetzt, die zu der Quervernetzung des Polymers führen.

An Stelle der inneren Gelierung kann auch ein anderes Verfahren zur Quervernetzung benutzt werden, z. B. die einfache Diffusion von Ca²⁺-Ionen aus einer Lösung oder eine kontrollierte Diffusion durch Membranen, wobei diese Aufzählung nicht erschöpfend ist.

Die erhaltenen Gele können getrocknet werden.
Die erhaltenen, gegebenenfalls getrockneten, Polymermatrizes können nach Bedarf in Stücke von für die jeweilige Anwendung geeigneter Größe geteilt werden. Die ggf. auf eine gewünschte Größe gebrachten Stücke können als Inserte eingesetzt werden, die in Abhängigkeit der ggf. enthaltenen Wirkstoffe geeignet verpackt lagerbar und unmittelbar einsetzbar sind.
Der Begriff "Insert" umfasst auch ein Implantat, das in den Körper eingebracht wird.

Die erfindungsgemäße Polymermatrix kann nach dem vorstehenden Verfahren auch unmittelbar am Applikationsort im Körper, in situ, erzeugt werden. Hierzu sollten die Reaktionsbedingungen vorzugsweise so gewählt werden, dass die Quervernetzung schnell erfolgt, zum Beispiel indem der pH-Wert der Reaktionslösungen entsprechend gewählt wird; im Falle von EDTA also im sauren Bereich.
Für die in situ Erzeugung können die Reaktionslösungen wie das unvernetzte gelbildende Polymer und Lösung mit quervernetzendem Agens, auf die Stelle, auf der die quervernetzte Polymermatrix ausgebildet werden soll, auf beliebige Weise aufgebracht werden wie zum Beispiel Aufspritzen, Aufsprühen, Aufpinseln etc.

Im Falle einer getrockneten Matrix wird diese bei Anwendung im menschlichen oder tierischen Körper durch Körperflüssigkeit (im Falle der Anwendung am Auge durch die Tränenflüssigkeit) rehydratisiert, wodurch sie leicht aufquillt. Beim physiologischen pH-Wert von 7,4 wird das in die Matrix inkorporierte Na₂-EDTA "aktiviert", das heißt die Komplexbildungskonstante wird erhöht, wodurch sich ein stabiler Ca-EDTA-Komplex ausbildet. Die quervernetzenden Ca²⁺-Ionen werden folglich der Matrix entzogen, so dass sich die Polymerketten langsam auflösen können.

Die Quellung des Polymernetzwerkes unterstützt, insbesondere auch bei anfänglicher räumlicher Trennung, die Diffusion des Komplexbildners zu den Bindungsstellen mit den mehrwertigen Kationen, um diese zu komplexieren. Auf diese Weise löst sich die Matrix innerhalb weniger Stunden in der sie umgebenden Körperflüssigkeit auf und muss nach der Applikation nicht mehr umständlich entfernt werden.

Die erfindungsgemäße Polymermatrix zeichnet sich insbesondere dadurch aus, dass sie bereits ein auflösendes Agens inkorporiert enthält. Nach Aktivierung des auflösenden Agens durch einen äußeren Stimulus bewirkt das auflösende Agens die Lösung der Bindung zwischen Bindungsstelle und quervernetzendem Agens, sodass sich die Polymermatrix auflösen kann.

Die Zeitdauer der Auflösung kann zum Beispiel durch die Art und/oder Menge an auflösenden Agens sowie die Art und Weise in der das Agens in der Matrix angeordnet ist, eingestellt und gesteuert werden.

Die erfindungsgemäße Polymermatrix eignet sich damit insbesondere zur Herstellung von Inserten, die sich nach Applikation im menschlichen oder tierischen Körper von selbst auflösen beziehungsweise von diesen resorbiert werden.

Der erfindungsgemäße Polymermatrix beziehungsweise die daraus erhaltenen Inserte können für die parenterale Applikation, zum Beispiel in Körperhöhlungen, oder zur lokalen Verabreichung von Wirkstoffen verwendet werden.
Ein Beispiel hierfür ist die lokale Anwendung am Auge. In diesem Fall kann die Aktivierung des auflösenden Agens durch die Tränenflüssigkeit erfolgen.

Ein weiteres Beispiel einer parenteralen Anwendung der erfindungsgemäßen Polymermatrix ist die Verwendung als Membran, insbesondere als Barrieremembran, zur Verringerung der postoperativen Narbenbildung nach zum Beispiel chirurgischen Eingriffen in oder am Körper.

Die postoperative Narbenbildung zwischen einer Operationswunde und dem angrenzenden Umgebungsgewebe, auch als Adhäsion bezeichnet, ist ein schwerwiegendes medizinisches Problem in der postoperativen Wundversorgung.
Erfindungsgemäß umfasst der Begriff "postoperative Narbenbildung" jede Form der Ausbildung von Narbengewebe wie zum Beispiel Adhäsion, und Bildung von Verwachsungen.

Narbenbildung oder Adhäsion ist ein Zustand, der die Bildung von anormalen Gewebeverbindungen umfasst. Diese Gewebeverbindungen wirken sich nachteilig auf Körperfunktionen aus, können den Darm und andere Teile des Gastrointestinaltrakts verschließen (Darmobstruktion), Unfruchtbarkeit und allgemeine Beschwerden, beispielsweise Beckenschmerzen, hervorrufen. Im schlimmsten Fall kann dieser Zustand lebensbedrohlich sein.

Die häufigste Form der Narbenbildung tritt nach einem chirurgischen Eingriff auf, jedoch auch in Folge anderer entzündlicher Prozesse oder Ereignisse wie mechanischen Verletzungen.

Zur Vermeidung beziehungsweise Verringerung der Narbenbildung ist es bekannt, die frisch operierten Stellen von angrenzendem Umgebungsgewebe durch Applikation von sogenannten Barrieremembranen räumlich zu trennen. Dadurch werden Zellmigration und Gefässeinwachsungen aus dem benachbarten Gewebe in die frischoperierte Stelle verhindert oder verlangsamt, sodass die Wundheilung ungestört verlaufen kann. Beispielsweise kann nach einem Eingriff in den Körper auf den frisch operierten Bereich eine Barrieremembran aufgelegt werden, die dann nach Wundverschluss die frisch operierte Stelle von darüber liegender Muskulatur abschirmt.

Ein Beispiel für eine derartige Barrieremembranen beschreibt EP 1 588 675 B1, wobei die Barrieremembran ein bioabbaubares Polylactidpolymer ist. Die Membran wird hier in vitro, außerhalb des Körpers, hergestellt und in eine geeignete Form gebracht und bei Bedarf appliziert.

Im Gegensatz dazu, kann die erfindungsgemäße Polymermatrix unmittelbar im Bereich der operierten Stelle in situ erhalten werden. Wie vorstehend ausgeführt, können hierzu die Reaktionslösungen zur Ausbildung eines quervernetzten Gels unmittelbar auf die gewünschte Stelle aufgebracht werden, zum Beispiel durch Spritzen, Aufsprühen, Aufpinseln etc.
Insbesondere können ihr auch gleichzeitig Wirkstoffe zugesetzt werden, die die Wundheilung fördern oder auf sonstige Weisung die Heilung unterstützen und beschleunigen.

Wie vorstehend beschrieben, kann die erfindungsgemäße Polymermatrix in für die jeweilige Applikation geeignete Dicke und Gestalt erhalten werden. Geeignete Dicken und Gestalten für den Einsatz als Barrieremembran können zum Beispiel der vorstehend genannten EP 1 588 675 B1 entnommen werden. Beispielsweise liegen geeignete Dicken in einem Bereich zwischen 10 µm und 300 µm und vorzugsweise zwischen 10 und 100 µm.

Für die Verwendung als Barrieremembran sollte der Auflösungszeitraum vorzugsweise zwischen 7 Tagen und 12 Wochen liegen. Wie bereits ausgeführt, lässt sich der Auflösungszeitraum auf unterschiedliche Weise einstellen, zum Beispiel durch Art des auflösenden Agens, Menge an auflösenden Agens, Verteilung in der Membran usw.
Insbesondere Membranen mit einem Auflösungszeitraum von mehr als 30 Tagen können auch als Softtissue Support eingesetzt werden.

### Beispiele zur Herstellung der beschriebenen Matrizes

### Beispiel 1: Verringerung der Komplexbildungskonstante

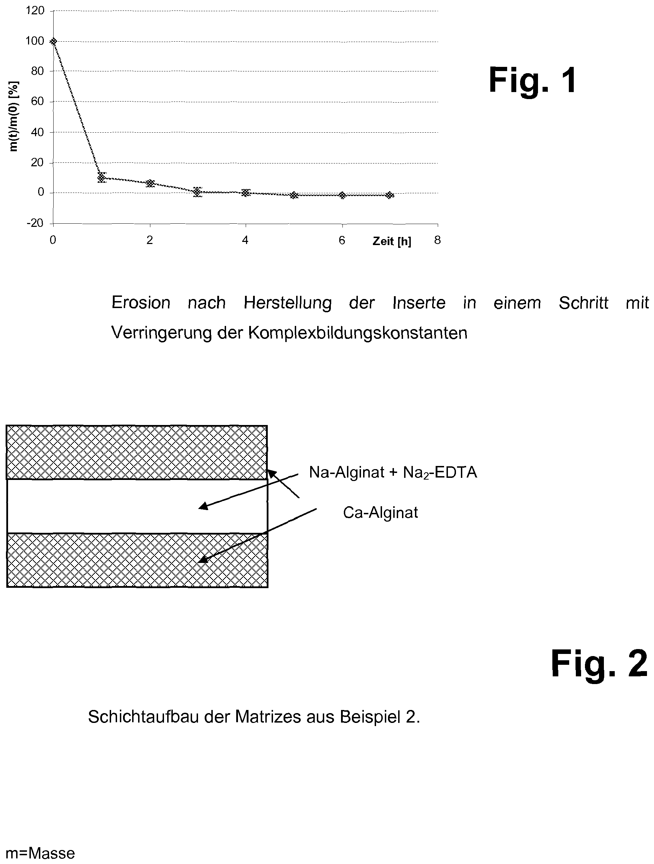

Die Matrix wurde aus Na-Alginat (65-75% Guluronsäure) in einem Schritt mittels innerer Quervernetzung hergestellt. Dazu wurden zunächst CaCl₂ · 2H₂O und Na₂EDTA · 2H₂O in äquimolarem Verhältnis in bidestilliertem Wasser gelöst und der pH-Wert der Lösung mit Natronlauge auf 7-7,5 eingestellt. Bei diesem pH-Wert ist der Ca-EDTA-Komplex am stabilsten. Das Natriumalginat wurde mit Glycerol gemischt und dann in der Ca-EDTA-Lösung gelöst (2% (m/V) Alginat, 5% (m/V) Glycerol). Zur dieser Lösung wurde 1% (m/V) Glucono-δ-lacton gegeben, welches in wässriger Lösung langsam zu Gluconsäure hydrolysiert und dadurch den pH-Wert verringert, wodurch der Ca-EDTA-Komplex instabiler wird. Damit die vorzeitige Hydrolyse zu Gluconsäure verhindert wird, wurde die Lösung sofort nach Zugabe des Lactons steril filtriert, und in eine Teflonschale gegossen und abgedeckt stehen gelassen, bis sich das Gel komplett ausgebildet hatte.
Danach wurde das Gel über Nacht bei Raumtemperatur getrocknet und anschließend Augeninserte von 5mm Durchmesser ausgestanzt.
Zur Überprüfung der Erosion wurden diese Inserte in 5 ml Tris-Puffer (pH 7,4) bei 37°C inkubiert; jede Stunde wurde der Zustand der Inserte makroskopisch überprüft und der Überstand abpipettiert. Nach Trocknung des Rückstands wurde dieser gewogen (n=3), um die Auflösung der Inserte graphisch darstellen zu können.
Der Versuch wurde über 8 Stunden durchgeführt.
Im Ergebnis erfolgte innerhalb der ersten Stunde ein Abbau um ca. 90%, mit vollständiger Auflösung nach ca. drei Stunden (s. Figur 1).

### Beispiel 2: Räumliche Trennung von Kationen und EDTA durch schichtweisen Aufbau der Matrices

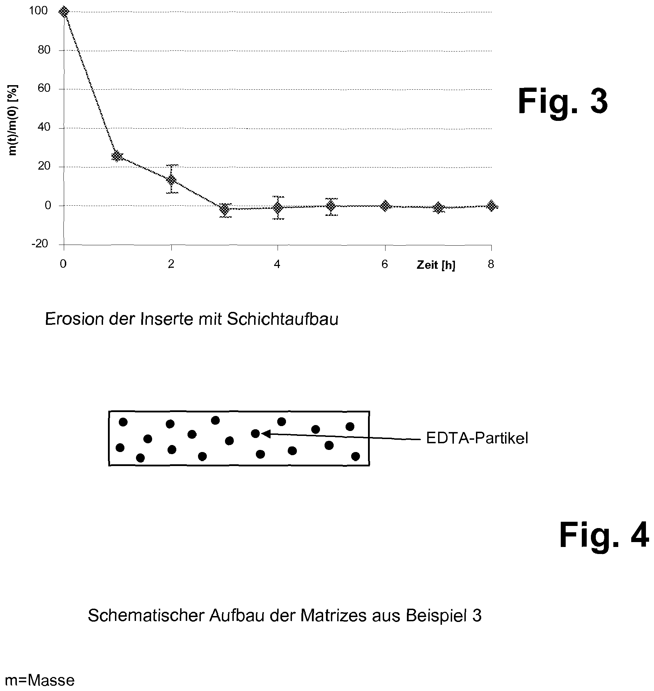

Zur räumlichen Trennung von Komplexbildner und Kationen wurde die Matrix aus drei Schichten aufgebaut (s. Figur 2) Zunächst wurde CaHPO₄ · 2H₂O in neutralem Puffer suspendiert und in dieser Suspension das Na-Alginat gelöst. Nach Zugabe von Glucono-δ-lacton wurde sterilfiltriert und die erste Schicht in einer Teflonschale zuerst gelieren und dann trocknen gelassen. Danach wurde eine sterilfiltrierte Na-Alginat-Lösung, die Na₂-EDTA enthielt, auf die getrocknete erste Schicht gegeben und ohne Quervernetzung trocknen gelassen. Die oberste Schicht wurde analog der ersten sterilfiltriert und aufgetragen.
Nachdem die Matrix komplett getrocknet war, wurden Augeninserte mit 5mm Durchmesser ausgestanzt und auf ihre Erosion untersucht. (s. Fig. 3).
Die beiden äußeren, quervernetzten Schichten verhindern das sofortige Lösen der Matrix. Erst nach erfolgter Quellung können sich diese beiden Schichten auflösen, sobald das freie Na₂-EDTA aus der mittleren Schicht die Ca²⁺-Ionen der beiden äußeren Schichten komplexiert.
Im Ergebnis zeigte sich, dass die vollständige Auflösung wie in Beispiel 1 gleichfalls nach ca. Stunden erfolgt war, jedoch betrug der Abbau nach einer Stunde nur etwa 75%.

### Beispiel 3: Räumliche Trennung von Kationen und EDTA durch Einsatz als Feststoff

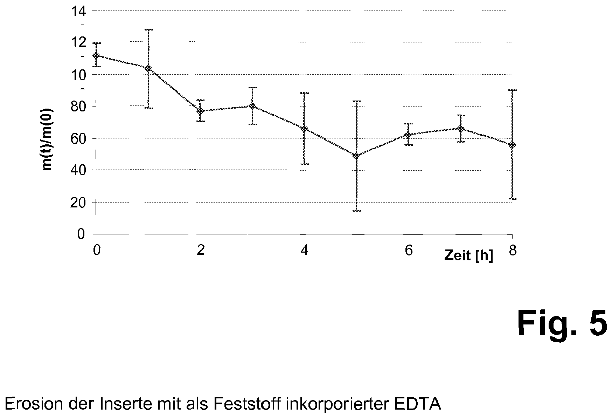

Diese Matrizes bestehen aus nur einer Schicht. Der Komplexbildner wurde hier als Feststoff eingearbeitet (s. Figur 4) und löst sich erst nach Kontakt mit Körperflüssigkeit langsam auf. Zunächst wurde eine EDTA-Suspension hergestellt, in der ein Na-Alginat mit geringerem Guluronsäureanteil (35-45 %) gelöst wurde. Diese dickflüssige Emulsion wurde zu einem Film gezogen, über Nacht getrocknet und anschließend durch einminütiges Einlegen in eine 5%ige CaCl₂-Lösung quervernetzt. Nachdem der Film wieder vollständig getrocknet war, wurden Augeninserte mit 5mm Durchmesser ausgestanzt.
Der Erosionsversuch wurde analog zu den anderen durchgeführt. Bei diesem Matrixaufbau zeigte sich allerdings, dass die Inserte sich nur sehr langsam lösten und nach 8 Stunden noch nicht komplett aufgelöst waren (s. Figur 5). Ein solcher Aufbau der beschriebenen Matrix eignet sich damit insbesondere für eine Langzeitanwendung.

## Patentansprüche

1. Polymermatrix mit der Fähigkeit zur kontrollierten Freisetzung eines darin inkorporierten Wirkstoffes, wobei die Polymermatrix aus gelbildenden Polymeren und einem quervernetzenden Agens gebildet ist, wobei die gelbildenden Polymere Bindungsstellen aufweisen, die mit dem quervernetzenden Agens unter Ausbildung von Quervernetzungen reagieren, wobei die Matrix zusätzlich auflösendes Agens enthält, das nach Aktivierung die Quervernetzung wieder löst und die Selbstauflösung der Polymermatrix bewirkt,
- **wobei** das auflösende Agens ein Komplexbildner ist, dessen Komplexbildungskonstante nach Aktivierung mit dem quervernetzenden Agens höher ist als die Komplexbildungskonstante der Bindungsstelle, **wobei** die Aktivierung durch die Umgebungsbedingungen am Applikationsort bewirkt wird, und
**wobei** im Verlauf der Selbstauflösung der Wirkstoff freigesetzt wird.

2. Polymermatrix nach Anspruch 1,
**wobei** die gelbildenden Polymere Polysaccharide sind.

3. Polymermatrix nach Anspruch 1 oder 2,
**wobei** das quervernetzende Agens mehrwertige Kationen sind.

4. Polymermatrix nach einem der vorhergehenden Ansprüche,
**wobei** das gelbildende Polysaccharid ein Alginat ist.

5. Polymermatrix nach einem der vorhergehenden Ansprüche,
**wobei** das auflösende Agens in der quervernetzten Matrix verteilt vorliegt.

6. Polymermatrix nach einem der Ansprüche 1 bis 4,
**wobei** die Matrix ein Schichtsystem ist, das aus mindestens zwei Schichten gebildet ist, wobei Schichten mit auflösenden Agens vorliegen und die Schichten mit auflösenden Agens optional nicht quervernetzt sind.

7. Polymermatrix nach Anspruch 6,
**wobei** eine nicht quervernetzte Schicht mit auflösenden Agens zwischen zwei quervernetzten Schichten ohne auflösenden Agens angeordnet ist.

8. Polymermatrix nach einem der vorhergehenden Ansprüche,
**wobei** das auflösende Agens durch Änderung des pH-Wertes aktiviert wird.

9. Polymermatrix nach einem der vorhergehenden Ansprüche,
**wobei** die Polymermatrix in getrockneter Form vorliegt.

10. Polymermatrix nach einem der vorhergehenden Ansprüche,
**wobei** die Polymermatrix einen Wirkstoff inkorporiert enthält.

11. Polymermatrix nach Anspruch 10,
**wobei** der Wirkstoff ein Arzneimittel ist.

12. Insert zur Applikation am und/oder in einem menschlichen oder tierischen Körper,
**wobei** das Insert aus einer Polymermatrix nach einem der Ansprüche 1 bis 11 gebildet ist.

13. Insert nach Anspruch 12,
**wobei** das auflösende Agens durch Kontakt der Polymermatrix mit einer Körperflüssigkeit aktiviert wird und das Insert sich auflöst.

14. Polymermatrix nach einem der Ansprüche 1 bis 11 oder eines Inserts nach einem der Ansprüche 12 und 13 zur Verwendung zur Applikation am Auge.

15. Polymermatrix nach einem der Ansprüche 1 bis 11 oder eines Inserts nach einem der Ansprüche 12 und 13 zur Verwendung für die parenterale Applikation.

16. Polymermatrix nach Anspruch 15 zur Verwendung als resorbierbare Membran zur Verminderung der Bildung von postoperativem Narbengewebe im Bereich einer postoperativen Stelle.

17. Polymermatrix nach Anspruch 16, wobei die Membran in situ erzeugt wird.

## Claims

1. Polymer matrix with the capability of controlled release of active substance incorporated therein,
**wherein** the polymer matrix is formed from gel-forming polymers and a cross-linking agent,
**wherein** the gel-forming polymers have binding sites that react with the cross-linking agent to form cross-links,
**wherein** the matrix additionally contains dissolving agent, which, after activation, breaks once again the cross-links and brings about the self-dissolution of the polymer matrix,
**wherein** the dissolving agent is a complex forming agent, the complex formation constant of which is higher after activation with the cross-linking agent than the complex formation constant of the binding site,
**wherein** activation is caused by the environmental conditions at the application site, and
**wherein** the active substance is released in the course of the self-dissolution.

2. The polymer matrix according to claim 1,
**wherein** the gel-forming polymers are polysaccharides.

3. The polymer matrix according to claim 1 or 2,
**wherein** polyvalent cations are the cross-linking agent.

4. The polymer matrix according to one of the preceding claims,
**wherein** the gel-forming polysaccharide is an alginate.

5. The polymer matrix according to one of the preceding claims,
**wherein** the dissolving agent is present in the cross-linking matrix in a distributed manner.

6. The polymer matrix according to one of claims 1 to 4,
**wherein** the matrix is a layer system, which is formed from at least two layers, with layers being present with dissolving agent and the layers with dissolving agent being optionally not cross-linked.

7. The polymer matrix according to claim 6,
**wherein** a non-cross-linked layer with dissolving agent is arranged between cross-linked layers without dissolving agent.

8. The polymer matrix according to one of the preceding claims,
**wherein** the dissolving agent is activated by change of the pH-value.

9. The polymer matrix according to one of the preceding claims,
**wherein** the polymer matrix is present in dried form.

10. The polymer matrix according to one of the preceding claims,
**wherein** the polymer matrix contains an incorporated active substance.

11. The polymer matrix according to claim 10,
**wherein** the active substance is a pharmaceutical.

12. An insert for application on and / or in a human or animal body,
**wherein** the insert is formed from a polymer matrix according to one of claims 1 to 11.

13. The insert according to claim 12,
**wherein** the dissolving agent is activated by contact of the polymer matrix with a body fluid and the insert dissolves.

14. The polymer matrix according to one of claims 1 to 11 or an insert according to one of claims 12 and 13 for utilization for application on the eye.

15. The polymer matrix according to one of claims 1 to 11 or an insert according to one of claims 12 and 13 for utilization for parenteral application.

16. The polymer matrix according to claim 15 for utilization as a resorbable membrane for reducing the formation of postoperative scar tissue in the region of a postoperative site.

17. The polymer matrix according to claim 16,
**wherein** the membrane is produced in situ.

## Revendications

1. Matrice polymère apte à la libération contrôlée d'un agent actif incorporé dans celle-ci, la matrice polymère étant formée par des polymères gélifiants et un agent réticulant, les polymères gélifiants comprenant des emplacements de liaison qui réagissent avec l'agent réticulant pour former des réticulations, la matrice contenant en outre un agent dissolvant, qui après activation redissout la réticulation et permet la dissolution automatique de la matrice polymère,
- dans laquelle l'agent dissolvant est un complexant, dont la constante de complexation après activation avec l'agent réticulant est plus élevée que la constante de réticulation de l'emplacement de liaison,
- dans laquelle l'activation est effectuée par les conditions de l'environnement à l'emplacement d'application, et
- dans laquelle l'agent actif est libéré au cours de la dissolution automatique.

2. Matrice polymère selon la revendication 1, dans laquelle les polymères gélifiants sont des polysaccharides.

3. Matrice polymère selon la revendication 1 ou 2, dans laquelle l'agent réticulant consiste en des cations polyvalents.

4. Matrice polymère selon l'une quelconque des revendications précédentes, dans laquelle le polysaccharide gélifiant est un alginate.

5. Matrice polymère selon l'une quelconque des revendications précédentes, dans laquelle l'agent dissolvant est présent sous forme répartie dans la matrice réticulée.

6. Matrice polymère selon l'une quelconque des revendications 1 à 4, dans laquelle la matrice est un système stratifié, qui est formé à partir d'au moins deux couches, des couches contenant l'agent dissolvant étant présentes et les couches contenant l'agent dissolvant n'étant optionnellement pas réticulées.

7. Matrice polymère selon la revendication 6, dans laquelle une couche non réticulée contenant l'agent dissolvant est agencée entre deux couches réticulées ne contenant pas l'agent dissolvant.

8. Matrice polymère selon l'une quelconque des revendications précédentes, dans laquelle l'agent dissolvant est activé par modification du pH.

9. Matrice polymère selon l'une quelconque des revendications précédentes, dans laquelle la matrice polymère se présente sous forme séchée.

10. Matrice polymère selon l'une quelconque des revendications précédentes, dans laquelle la matrice polymère contient un agent actif incorporé.

11. Matrice polymère selon la revendication 10, dans laquelle l'agent actif est un médicament.

12. Insert destiné à être appliqué sur et/ou dans un corps humain ou animal, l'insert étant formé à partir d'une matrice polymère selon l'une quelconque des revendications 1 à 11.

13. Insert selon la revendication 12, dans lequel l'agent dissolvant est activé par contact de la matrice polymère avec un fluide corporel et l'insert se dissout.

14. Matrice polymère selon l'une quelconque des revendications 1 à 11 ou insert selon l'une quelconque des revendications 12 et 13, destiné à une utilisation pour l'application sur l'oeil.

15. Matrice polymère selon l'une quelconque des revendications 1 à 11 ou insert selon l'une quelconque des revendications 12 et 13, destiné à une utilisation pour l'application parentérale.

16. Matrice polymère selon la revendication 15, destinée à une utilisation en tant que membrane résorbable pour réduire la formation de tissus cicatriciels postopératoires dans la zone d'un emplacement postopératoire.

17. Matrice polymère selon la revendication 16, dans laquelle la membrane est générée in situ.
